# EUROPEAN PATENT APPLICATION

(11) **EP 4 740 941 A1**
(43) Date of publication of application: **13.05.2026**
(21) Application number: 24836380.6
(22) Date of filing: 05.07.2024
(51) Int. Cl.: A61K 31/137, A61P 17/18, A61P 39/06

(54) **COMPOSITION FOR DELAYING AGING OR EXTENDING LIFESPAN COMPRISING AMINOAROMATIC COMPOUND AS ACTIVE INGREDIENT**

(30) Priority: 05.07.2023 KR 20230087239; 04.07.2024 KR 20240088444
(71) Applicant: Institute for Basic Science, Yuseong-gu Daejeon 34126 (KR)
(72) Inventor: PARK, Ki Duk, Seoul 02792 (KR); PARK, Jong Hyun, Seoul 02792 (KR); CHOI, Ji Won, Seoul 02792 (KR); LEE, Elijah, Seoul 02792 (KR); LEE, Changjoon, Daejeon 34125 (KR); WON, Woojin, Seoul 04385 (KR); MRIDULA, Bhalla, Daejeon 34126 (KR)
(74) Representative: Kraus & Lederer PartGmbB
(86) International application number: PCT/KR2024/009597
(87) International publication number: WO 2025/009933

(57) **Abstract**

The present invention provides: a pharmaceutical composition for preventing or treating aging-related diseases or disorders, comprising an aminoaromatic compound or a pharmaceutically acceptable salt thereof as an active ingredient; a composition for delaying aging or extending lifespan; and a health functional food composition for preventing or alleviating aging-related diseases.

## Description

### [Technical Field]

The present invention relates to a composition for delaying aging or extending lifespan including an aminoaromatic compound as an effective component.

### [Background Art]

Living things have a limited lifespan and naturally go through a process of being born, growing up, and growing old, that is, aging over time and ultimately leading to death. Aging is one of the most complex biological pathways which occurs in all living organisms.

In order to reveal the cause and process of the aging, many studies have been conducted, and an aging phenomenon is largely explained by two mechanisms of aging clock and cellular aging. The aging clock is a theory explained by the loss of telomere replication at the end of DNA, and states that the depletion of telomere which is a repetitive sequence found at both ends of each chromosome may lead to cellular aging and organism aging. In the cellular aging, accumulation of intracellular damage due to oxidative damage, mitochondrial dysfunction, and the like is considered to be the main cause of aging, and among them, a mitochondrial decline theory emphasizes the importance of mitochondrial function in healthy cell metabolism and bioenergy. A decline in mitochondrial function related to aging includes a decrease in ATP production, an increase in ROS production, a decreased in the number of mitochondria, and a change in mitochondrial permeability.

As aging progresses, activity of pathways involved in fat metabolism is impaired. That is, lipolysis is decreased, and absorption of low-density cholesterol (LDL) in cells is decreased to increase LDL concentration in blood, which may be the cause of cardiovascular diseases. In addition, aging causes a decline in cognitive and motor functions and an increase in the incidence of neurological diseases, such as Alzheimer's disease and Parkinson's disease, which reduce quality of life.

In the aging process, various physiological changes such as decreased body and bone mass, increased blood pressure and blood sugar, and decreased exercise amount and cardiac output appear. At a molecular level, aging is characterized by DNA mutation, protein oxidation, and lipid peroxidation accumulation, but a fundamental mechanism which may explain the changes related to aging and increased mortality has not yet been fully revealed.

In addition to the study focused on understanding the aging process, many studies for delaying aging and extending lifespan are also being conducted. The most successful method so far is dietary restriction. The effect of dietary restriction was first reported in rats, and then a longevity phenotype due to dietary restriction was observed in yeast, nematodes, fruit flies, and mice. However, the beneficial effect of the dietary restriction as described above is observed only when the dietary restriction is continuously maintained, and it is difficult to apply dietary restriction to all living things.

Various studies and efforts have been made to date in order to achieve the dream of immortality, but the aging mechanism has yet to be fully elucidated.

Meanwhile, it was reported that an aminoaromatic compound, which is a blood hydrogen peroxide scavenger, eliminates a significantly high level of hydrogen peroxide present in pathological conditions such as neurodegenerative diseases, in particular, Alzheimer's disease to an appropriate level of concentration, and is effective in treating neurodegenerative diseases. However, the effect of elimination of excessive hydrogen peroxide from the blood on biological aging, particularly in lifespan extension and aging delay, have not been studied.

### [Disclosure]

### [Technical Problem]

The present inventors confirmed that an aminoaromatic compound known as a blood hydrogen peroxide scavenger may delay biological aging and extend lifespan and were inspired to apply it as a use related to aging delay or life extension, thereby completing the present invention.

An objective of the present invention is to provide a pharmaceutical composition for preventing or treating age-related diseases or disorders including an aminoaromatic compound or a pharmaceutically acceptable salt thereof as an effective component.

Another objective of the present invention is to provide a composition for delaying aging or extending lifespan including an aminoaromatic compound or a pharmaceutically acceptable salt thereof as an effective component.

Still another objective of the present invention is to provide a health functional food composition for preventing or alleviating age-related diseases including an aminoaromatic compound or a food-scientifically acceptable salt thereof as an effective component.

### [Technical Solution]

In one general aspect,
a pharmaceutical composition for preventing or treating age-related diseases or disorders includes: an aminoaromatic compound represented by the following Chemical Formula 1 or a pharmaceutically acceptable salt thereof as an effective component: 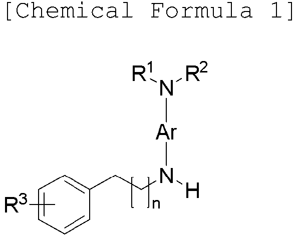
wherein
Ar is C₆-C₂₀ arylene, and the arylene of Ar may be further substituted by one or more selected from C₁-C₁₀ alkyl, C₁-C₁₀ alkoxy, amino, mono- or di-C₁-C₁₀ alkylamino, haloC₁-C₁₀ alkyl, haloC₁-C₁₀ alkoxy, and hydroxy;
R¹ and R² are independently of each other hydrogen or C₁-C₁₀ alkyl;
R³ is halogen, C₁-C₁₀ alkoxy, haloC₁-C₁₀ alkyl, or haloC₁-C₁₀ alkoxy; and
n is an integer of 1 or 2,
provided that when R³ is halogen, n is an integer of 1.

In another general aspect, a composition for delaying aging or extending lifespan includes: an aminoaromatic compound represented by Chemical Formula 1 or a pharmaceutically acceptable salt thereof as an effective component.

In still another aspect, a health functional food composition for preventing or alleviating age-related diseases includes: an aminoaromatic compound represented by Chemical Formula 1 or a food-scientifically acceptable salt thereof as an effective component.

### [Advantageous Effects]

The composition according to an exemplary embodiment may delay age-related neurodegeneration by significantly inhibiting neuronal cell death due to aging and may significantly improve a degree of decline in motor function involved in aging by improving the survival rate of dopamine-secreting neurons.

In addition, the size and number of branches of astrocytes tend to decrease as aging progresses, but this may be delayed by the treatment or administration of the composition according to an exemplary embodiment. In addition, as aging progresses, reactive oxygen and GABA which cause memory loss or cognitive impairment due to the overproduction of MAOB in astrocytes are overproduced, but reactive oxygen and GABA overproduction may be inhibited by the treatment or administration of the composition according to an exemplary embodiment.

That is, the composition according to an exemplary embodiment may delay the aging of an individual to extend lifespan and may prevent or treat diseases involved in aging or disorders. Besides, the composition may show preventive or restorative effects on decline in motor function due to aging.

### [Description of Drawings]

FIG. 1 shows the results of the survival rate of C57BL/6 female mice depending on whether an aminoaromatic compound was administered, the dosage and the administration period in Example 1 (*p=0.0126, control group to KDS12025 0.1 mpk; #p=0.0142, control group to KDS12025 1 mpk, $p=0.0306, Mantel-Cox assay).
FIG. 2 shows the results of an open field (OF) test in Example 2.
FIG. 3 shows the results of a novel place recognition (NPR) test in Example 2.
FIG. 4 shows the results of Y-maze examination in Example 2.
FIG. 5 shows the results of elevated plus maze in Example 2.
FIG. 6 shows the immunohistochemical staining photographs and results of the hippocampi of 18- and 30-month-old mice; the results of neuronal cell death and inhibition.
FIG. 7 shows the immunohistochemical staining photographs and results of the substantia nigra of a 30-month-old mice; the results of dopamine neuronal cell death and inhibition.
FIG. 8 is the immunohistochemical staining photographs of the hippocampi of 18- and 30-month-old mice.
FIG. 9 shows immunohistochemical staining results; a measurement indicator 1 of an aging degree of astrocytes.
FIG. 10 is the morphological analysis and photographs of astrocytes of the hippocampi of 18- and 30-month-old mice.
FIG. 11 shows immunohistochemical staining results; a measurement indicator 2 of an aging degree of astrocytes.
FIG. 12 shows immunohistochemical staining results; a measurement indicator 3 of an aging degree of astrocytes.
FIG. 13 shows the immunohistochemical staining photographs and results of the hippocampi of 18- and 30-month-old mice; the measurement of monoamine oxidase (MAOB) protein which is one of the pathological indicators of astrocytes.

### [Best Mode]

Hereinafter, the present invention will be described in detail. Here, technical terms and scientific terms used in the present specification have the general meaning understood by those skilled in the art to which the present invention pertains unless otherwise defined, and description of the known function and configuration which may unnecessarily obscure the gist of the present invention will be omitted in the following description.

The following terms used in the present specification are defined as follows, but they are only illustrative and do not limit the present invention, application, or use.

The terms "substituent", "radical", "group", and "moiety" may be used interchangeably.

The term "C_{A}-C_{B}" refers to "having A or more and B or less carbon atoms".

The term "alkyl" refers to a monovalent straight-chain or branched chain saturated hydrocarbon radical composed of only carbon and hydrogen atoms. The alkyl may have 1 to 10, 1 to 7, or 1 to 4 carbon atoms. "Lower alkyl" refers to straight chain or branched chain alkyl having 1 to 4 carbon atoms. As an example, the alkyl includes methyl, ethyl, propyl, isopropyl, butyl, isobutyl, t-butyl, pentyl, hexyl, ethylhexyl, and the like, but is not limited thereto.

The term "arylene" refers to a divalent organic radical of an aromatic ring derived from aromatic hydrocarbon by removal of two hydrogen atoms, including a single- or fused ring system containing appropriately 4 to 7, preferably 5 or 6 ring atoms in each ring, and even a form in which a plurality of aryls are connected by a single bond. A specific example thereof includes phenylene, naphthylene, biphenylene, anthrylene, and the like, but is not limited thereto.

The term "alkoxy" is a -O-alkyl radical in which "alkyl" is as defined above. A specific example thereof includes methoxy, ethoxy, isopropoxy, butoxy, isobutoxy, t-butoxy, and the like, but is not limited thereto.

The term "halo" or "halogen" refers to a halogen group element, and includes, for example, fluoro, chloro, bromo, and iodo.

The term "haloalkyl" or "haloalkoxy" refers to an alkyl or alkoxy group in which one or more hydrogen atoms are substituted by a halogen atom, respectively, in which alkyl and halogen are as defined above. For example, haloalkyl may be fluoromethyl, difluoromethyl, trifluoromethyl, fluoroethyl, difluoroethyl, perfluoroethyl, and the like, and haloalkoxy may be fluoromethoxy, difluoromethoxy, trifluoromethoxy, fluoroethoxy, difluoroethoxy, perfluoroethoxy, and the like.

The term "amino" refers to -NH₂, and "hydroxy" refers of -OH.

The term "alkylamino" refers to an amino radical in which one or two alkyls are substituted, and a specific example thereof includes methylamino (-NHMe), dimethylamino (-NMe₂), ethylamino (-NHEt), diethylamino (-NEt₂), and the like, but is not limited thereto.

The term "pharmaceutically acceptable" represents a characteristic of being non-toxic to individuals such as cells or humans exposed to the composition, means that it is appropriate for use as a pharmaceutical preparation, is generally regarded as being safe for the use, and means that it is officially approved by the national management agency or listed in the Korean Pharmacopoeia or the US Pharmacopoeia.

The term "pharmaceutically acceptable salt" refers to any organic or inorganic addition salt of the compound of the present invention, in which the side effects caused by the salt does not reduce advantageous efficacy of the compound of the present invention itself at a concentration having a relatively non-toxic and harmless effective action to a patient.

The term "pharmaceutically acceptable excipient" and "pharmaceutically acceptable carrier" refer to materials which aid administration of an activator and absorption by a subject.

The term "prevention" refers to all actions which inhibit or delay occurrence, spread, and recurrence of age-related diseases or disorders.

The term "amelioration" refers to all actions of at least decreasing parameters related to the conditions to be treated, for example, severity of symptoms.

The term "treatment" refers to all actions of improving or beneficially changing the symptoms of age-related diseases or disorders.

The term "individual" refers to all animals including humans having developed or being likely to develop age-related diseases or disorders, or in need of age delay or life extension. The animals may be mammals such as cattle, horses, sheep, pigs, goats, camels, antelopes, dogs, and cats in need of treatment of similar symptoms, as well as humans, but are not limited thereto.

The term "administration" means that the pharmaceutical composition of the present invention is introduced to an individual by any appropriate method, and the administration route of the composition of the present invention may be various, such as oral or parenteral, as long as the composition may reach a target tissue.

The term "pharmaceutically effective amount" refers to an amount which is sufficient to treat a disease at a reasonable benefit/risk ratio applicable to medical treatment and does not cause side effects, and an effective dose level may be easily determined by a person skilled in the art according to factors including patients' gender, age, weight, and health state, the kind of diseases, severity, an activity of a drug, a sensitivity to a drug, an administration manner, an administration time, administration route and releasing rate, a treatment period, formulation, or a simultaneously used drug, and other factors well known in the medical field.

The term "food" may be meat, sausage, bread, chocolate, candy, snacks, confectionery, pizza, ramen, other noodles, chewing gum, dairy products including ice cream, various soups, beverages, tea, drinks, alcoholic beverages, vitamin complexes, health functional food, health food, and the like, and includes all foods in a common sense.

The term "health functional food" refers to food manufactured and processed using raw materials or components having functionality useful to the human body in accordance with Functional Foods for Health Act No. 6727, and "functional" refers to regulating nutrients for the structure and function of the human body or intake for the purpose of obtaining a beneficial effect for health applications such as physiological action.

The term "food-scientifically acceptable salt" refers to a formulation of a compound, which does not cause serious irritation to an organism to which a compound is administered and does not damage the biological activity and physical properties of the compound.

The present invention provides a pharmaceutical composition for preventing or treating age-related diseases or disorders including: an aminoaromatic compound represented by the following Chemical Formula 1 or a pharmaceutically acceptable salt thereof as an effective component:

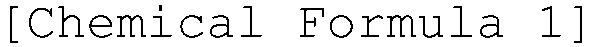

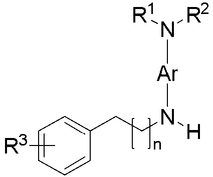

wherein
Ar is C₆-C₂₀ arylene, and the arylene of Ar may be further substituted by one or more selected from C₁-C₁₀ alkyl, C₁-C₁₀ alkoxy, amino, mono- or di-C₁-C₁₀ alkylamino, haloC₁-C₁₀ alkyl, haloC₁-C₁₀ alkoxy, and hydroxy;
R¹ and R² are independently of each other hydrogen or C₁-C₁₀ alkyl;
R³ is halogen, C₁-C₁₀ alkoxy, haloC₁-C₁₀ alkyl, or haloC₁-C₁₀ alkoxy; and
n is an integer of 1 or 2,
provided that when R³ is halogen, n is an integer of 1.

In addition, the present invention provides a composition for delaying aging or extending lifespan including: an aminoaromatic compound represented by Chemical Formula 1 or a pharmaceutically acceptable salt thereof as an effective component.

The aminoaromatic compound is a compound described in Korean Patent Registration No. KR 10-2643653 B1 issued to the present application and is known to act as a scavenger which removes hydrogen peroxide as one type of reactive oxygen and being useful for prevention, amelioration, or treatment of neurodegenerative diseases.

According to an exemplary embodiment of the present invention, the aminoaromatic compound may extend lifespan by effectively delaying aging of an individual and may be useful for the prevention or treatment of age-related diseases.

The term "aging" refers to a phenomenon involving various physiological changes such as decreased homeostasis of organs and organ systems and increased susceptibility to external stress and diseases as cell differentiation and proliferation decrease and function deteriorates over time since the birth of an individual, and the term "age-related disease" refers to a disease which occurs more frequently with aging.

In the present invention, delaying aging of an individual refers to progressing aging more slowly by decreasing the speed of aging occurring over time as compared with a control group, that is, an individual which is not treated or administered with the composition according to an exemplary embodiment or the compound. In addition, in the present invention, extending the life of an individual refers to longer survival of an individual as compared with a negative control group, that is, an individual which is not treated or administered with the composition according to an exemplary embodiment or the compound.

In an exemplary embodiment, the age-related disease may be one or more diseases selected from the group consisting of Alzheimer's disease, diabetes, Parkinson's disease, Huntington's disease, degenerative joint disease, stroke, angina pectoris, osteoporosis, myocardial infarction, muscular dystrophy, amyotrophic lateral sclerosis, sarcopenia, liver cirrhosis, and chronic kidney disease.

In an exemplary embodiment, the age-related disease may be a cognitive dysfunction disease due to aging, and the cognitive function may be specifically one or more selected from the group consisting of perception, memory, attention, speech comprehension, speech generation, reading comprehension, creation of imagery, learning, and reasoning.

The composition according to an exemplary embodiment may delay age-related neurodegeneration by significantly inhibiting neuronal cell death due to aging and may significantly improve a degree of decline in motor function involved in aging by improving the survival rate of dopamine-secreting neurons.

In addition, the size and number of branches of astrocytes tend to decrease as aging progresses, but this may be delayed by the treatment or administration of the composition according to an exemplary embodiment. In addition, as aging progresses, MAOB in astrocytes which causes memory loss or cognitive impairment due to the overproduction of GABA, is overproduced, but GABA overproduction may be prevented or reduced by the treatment or administration of the composition according to an exemplary embodiment.

That is, the composition according to an exemplary embodiment may delay the aging of an individual to extend lifespan and may prevent or treat diseases involved in aging or disorders. Besides, the composition may show preventive or restorative effects on a decline in motor function due to aging.

In Chemical Formula 1, Ar may be C₆-C₁₂ arylene, preferably phenylene or biphenylene; and Ar may be further substituted by one or more selected from C₁-C₇ alkyl, C₁-C₇ alkoxy, amino, and hydroxy.

In Chemical Formula 1, Ar may be phenylene, and preferably, a nitrogen atom may be introduced the 1- and 4-positions of the phenylene, respectively.

The aminoaromatic compound may be specifically represented by the following Chemical Formula 2 or 3:

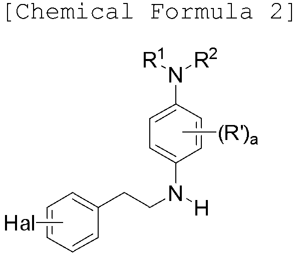

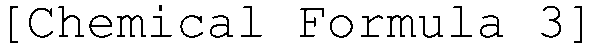

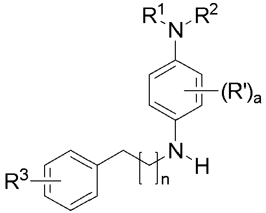

wherein
R¹ and R² are independently of each other hydrogen or C₁-C₇ alkyl;
Hal is halogen;
R³ is C₁-C₇ alkoxy or haloC₁-C₇ alkyl;
R' is C₁-C₇ alkyl, C₁-C₇ alkoxy, amino, or hydroxy;
a is an integer of 0 to 4; and
n is an integer of 1 or 2.

In Chemical Formulae 2 and 3, R¹ and R² may be independently of each other hydrogen or C₁-C₄ alkyl; Hal may be a halogen; R³ may be C1-C4 alkoxy or haloC1-C4 alkyl; a may be an integer of 0; and n may be an integer of 1 or 2.

Chemical Formula 2 may be represented by the following Chemical Formula 4:

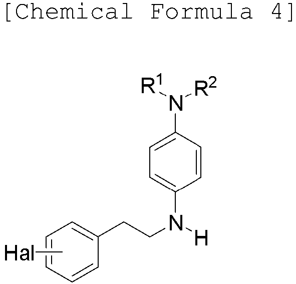

wherein
R¹ and R² are independently of each other hydrogen or C₁-C₄ alkyl; and
Hal is halogen.

Specifically, in Chemical Formula 4, R¹ and R² may be independently of each other C₁-C₄ alkyl; and Hal may be halogen.

Specifically, in Chemical Formula 4, R¹ may be hydrogen; R² may be C₁-C₄ alkyl; and Hal may be halogen.

Specifically, in Chemical Formula 4, R¹ and R² may be independently of each other hydrogen; Hal may be halogen.

Chemical Formula 3 may be represented by the following Chemical Formula 5:

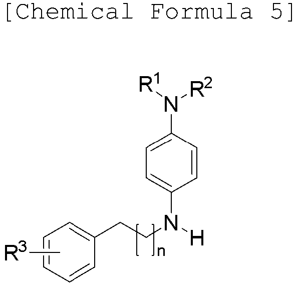

wherein
R¹ and R² are independently of each other hydrogen or C₁-C₄ alkyl;
R³ is C₁-C₄ alkoxy or haloC₁-C₄ alkyl; and
n is an integer of 1 or 2.

Specifically, in Chemical Formula 5, R¹ and R² may be independently of each other C₁-C₄ alkyl; R³ may be C₁-C₄ alkoxy or haloC₁-C₄ alkyl; and n may be an integer of 1 or 2.

Specifically, in Chemical Formula 5, R¹ may be hydrogen; R² may be C₁-C₄ alkyl; R³ may be C₁-C₄ alkoxy or haloC₁-C₄ alkyl; and n may be an integer of 1 or 2.

Specifically, in Chemical Formula 5, R¹ and R² may be independently of each other hydrogen; R³ may be haloC₁-C₄ alkyl; and n may be an integer of 1 or 2.

In any compound described in the present specification, halogen or halo may be fluorine.

The aminoaromatic compound may be any one selected from the following compound group, and is not limited thereto:

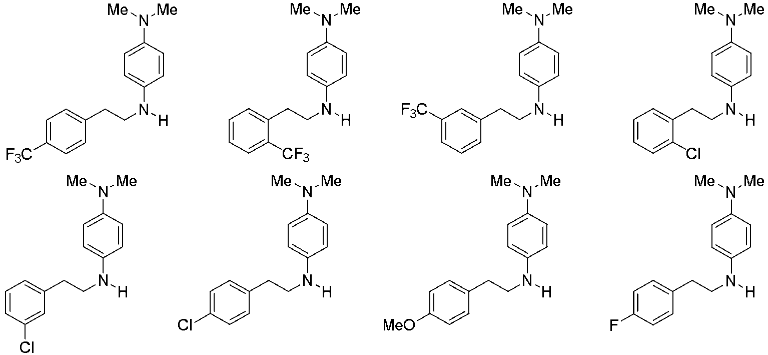

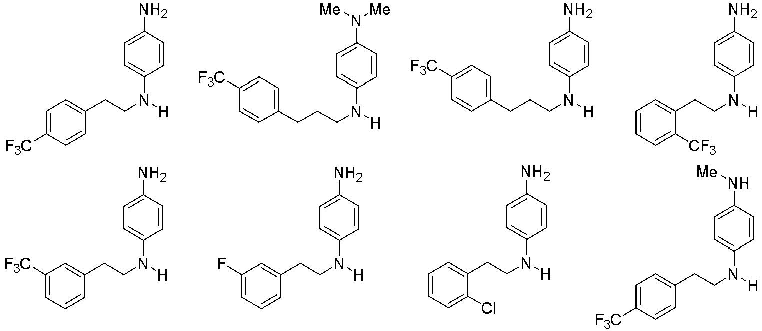

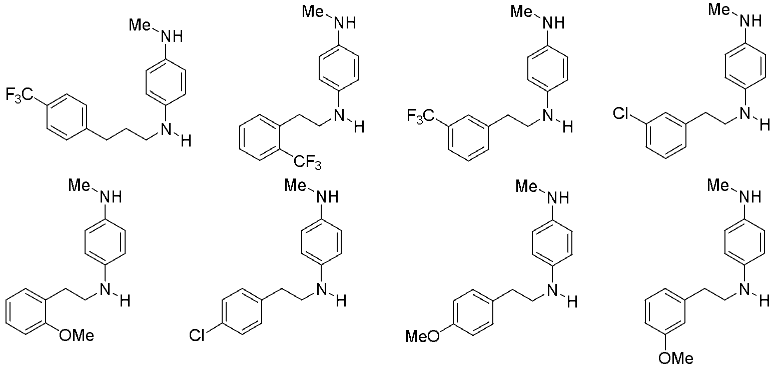

The aminoaromatic compound may be used in the form of a pharmaceutically acceptable salt, and the pharmaceutically acceptable salt is a salt prepared according to a common method in the art, and the preparation method thereof is known to a person skilled in the art. Specifically, the pharmaceutically acceptable salt includes salts derived from the following free acids and bases which are pharmacologically or physiologically acceptable, but is not limited thereto.

An acid addition salt formed by the pharmaceutically acceptable free acid is obtained from inorganic acids such as hydrochloric acid, nitric acid, phosphoric acid, sulfuric acid, hydrobromic acid, hydroiodic acid, nitrous acid, and phosphorous acid, and organic acids such as methanesulfonic acid, p-toluenesulfonic acid, acetic acid, trifluoroacetic acid, maleic acid, succinic acid, oxalic acid, benzoic acid, tartaric acid, fumaric acid, mandelic acid, propionic acid, citric acid, lactic acid, glycolic acid, gluconic acid, galacturonic acid, glutamic acid, glutaric acid, glucuronic acid, aspartic acid, ascorbic acid, carbonic acid, vanillic acid, and hydroiodic acid. The kind of pharmaceutically non-toxic salts as such includes sulfate, pyrosulfate, bisulfate, sulfite, bisulfite, nitrate, phosphate, monohydrogen phosphate, dihydrogen phosphate, metaphosphate, pyrophosphate chloride, bromide, iodide, fluoride, acetate , propionate, decanoate, caprylate, acrylate, formate, isobutyrate, caprate, heptanoate, propiolate, oxalate, malonate, succinate, suberate, sebacate, fumarate, maleate, butyne-1,4-dioate, hexane-1,6-dioate, benzoate, chlorobenzoate, methylbenzoate, dinitrobenzoate, hydroxybenzoate, methoxybenzoate, phthalate, terephthalate, benzenesulfonate, toluenesulfonate, chlorobenzenesulfonate, xylenesulfonate, phenylacetate, phenylpropionate, phenylbutyrate, citrate, lactate, β-hydroxybutyrate, glycolate, malate, tartrate, methanesulfonate, propanesulfonate, naphthalene-1-sulfonate, naphthalene-2-sulfonate, mandelate, and the like.

The acid addition salt may be prepared by a common method, and for example, may be prepared by dissolving the aminoaromatic compound in a water-miscible organic solvent such as methanol, ethanol, acetone, dichloromethane, and acetonitrile, adding an organic acid or inorganic acid to produce a precipitate, and filtering and drying the precipitate, or by distilling a solvent and an excessive amount of acid under reduced pressure, performing drying, and performing crystallization under an organic solvent.

In addition, a pharmaceutically acceptable metal salt may be prepared using a base. An alkali metal salt or an alkaline earth metal salt is obtained by, for example, dissolving the aminoaromatic compound in an excessive amount of an alkali metal hydroxide or alkaline earth metal hydroxide solution, filtering the undissolved aminoaromatic compound, and then evaporating and drying a filtrate. Herein, it is pharmaceutically appropriate to prepare a sodium, potassium, or calcium salt as a metal salt, but which is not limited thereto. In addition, a silver salt corresponding thereto may be obtained by reacting the alkali metal or alkaline earth metal salt with an appropriate silver salt (e.g., silver nitrate).

Preferably, the pharmaceutically acceptable salt of the aminoaromatic compound may be a hydrochloride.

That is, the aminoaromatic compound may be a compound in the form of a hydrochloride selected from the following structures:

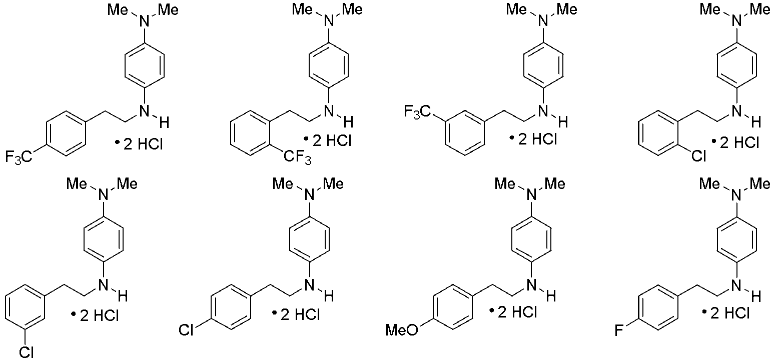

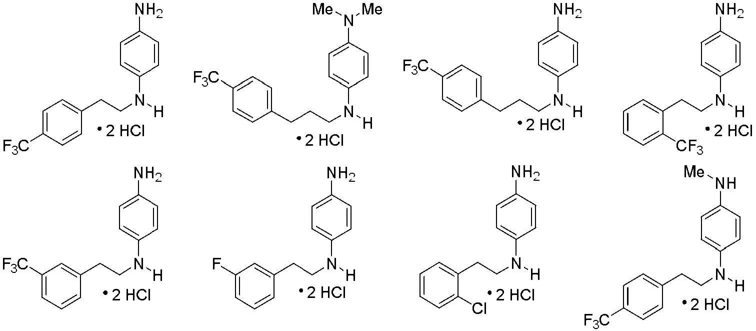

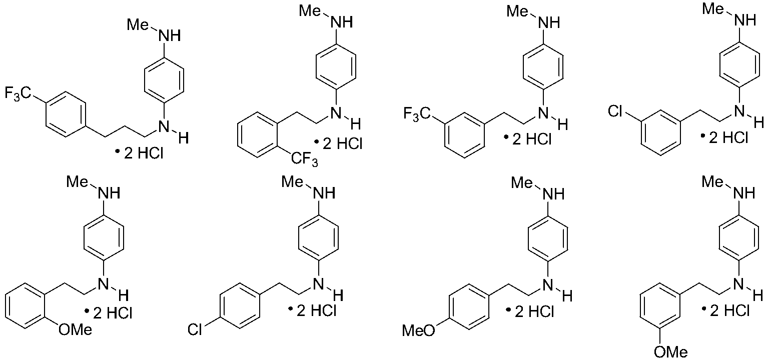

The composition according to an exemplary embodiment further includes a common non-toxic pharmaceutically acceptable carrier and/or excipient in addition to the effective component and may be formulated into a common preparation in the pharmaceutical field, that is, a preparation for oral administration or a preparation for parenteral administration. In addition, a diluent such as a filler, an extender, a binder, a wetting agent, a disintegrant, and a surfactant may be further included.

The pharmaceutically acceptable carrier, excipient, or diluent may be lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate, mineral oil, and the like, but is not limited thereto.

The composition according to an exemplary embodiment may be formulated into various forms, for example, oral formulations such as powders, granules, tablets, capsules, suspensions, emulsions, syrups, and aerosols, injections of sterile injection solutions, and the like by a common method according to the purpose of use, and may be orally administered or administered by various routes including intravenous, intraperitoneal, subcutaneous, rectal, and topical administrations.

The composition according to an exemplary embodiment may further include a filler, an anticoagulant, a lubricant, a wetting agent, a flavoring, an emulsifying agent, an antiseptic agent, and the like.

An example of a formulation for oral administration may include tablets, pills, hard/soft capsules, solutions, suspensions, emulsions, syrups, granules, elixirs, and the like, and these formulations may use one or more of commonly used diluents or excipients such as fillers, extenders, wetting agents, disintegrants, lubricants, binders, and surfactants, in addition to the effective component. As a disintegrant, agar, starch, alginic acid or a sodium salt thereof, an anhydrous calcium monohydrogen phosphate salt, and the like may be used, as a lubricant, silica, talc, stearic acid, or a magnesium salt or calcium salt thereof, polyethylene glycol, and the like may be used, and as a binder, magnesium aluminum silicate, starch paste, gelatin, tragacanth, methyl cellulose, sodium carboxymethyl cellulose, polyvinylpyrrolidone, low-substituted hydroxypropyl cellulose, and the like may be used. Other than that, lactose, dextrose, sucrose, mannitol, sorbitol, cellulose, glycine, and the like may be used as a diluent, and if necessary, commonly known effervescent mixtures, absorbents, colorants, flavoring agents, sweetening agents, and the like may be used therewith.

An example of a preparation for parenteral administration may include sterile aqueous solutions, nonaqueous solvents, suspensions, emulsions, freeze-dried preparations, suppositories, and the like. As the nonaqueous solvent and the suspension, propylene glycol, polyethylene glycol, a vegetable oil such as an olive oil, an injectable ester such as ethyloleate, and the like may be used. As a base of the suppository, witepsol, macrogol, tween 61, cacao butter, laurin butter, glycerol, gelatin, and the like may be used. Meanwhile, an injection may include a conventional additive such as a solubilizer, an isotonic agent, a suspending agent, an emulsifying agent, a stabilizer, and an antiseptic agent. For formulation into the injection, the aminoaromatic compound of the present invention or the pharmaceutically acceptable salt thereof is mixed in water with a stabilizer or a buffer to prepare a solution or a suspension, which may be produced into a unit dosage form of an ampule or vial.

The composition according to an exemplary embodiment may be sterilized, may further include an adjuvant such as an antiseptic agent, a stabilizer, a thickener, a hydrating agent or an emulsifying accelerator, a salt for regulating osmotic pressure, and/or a buffer, or may further include other therapeutically useful materials, and may be formulated according to a conventional method such as dissolution, dispersion, mixing, granulation, gelling, or coating.

The pharmaceutically effective amount of the aminoaromatic compound may be determined by factors including the health state of a patient, the kind of diseases, severity, an activity of a drug, a sensitivity to a drug, an administration manner, an administration time, administration route and releasing rate, a treatment period, combination, and a simultaneously used drug, and other factors well known in the medical field. Specifically, the effective amount of the aminoaromatic compound in the composition according to an exemplary embodiment may vary depending on the age, the gender, and the weight of a patient, and generally, about 0.01 to 500 mg/kg/day, preferably 0.1 to 100 mg/kg/day may be administered every day or every other day or administered in a divided dose of once or several times a day. However, since the amount may be increased or decreased depending on an administration route, severity of the disease, gender, weight, age, and the like, the administration amount does not limit the scope of the present invention in any way.

The composition according to an exemplary embodiment may be orally or parenterally administered, and parenteral administration such as subcutaneous, intravenous, intramuscular, or intraperitoneal injection is preferred.

The composition according to an exemplary embodiment may be administered as an individual therapeutic agent or in combination with other therapeutic agents, may be sequentially or simultaneously administered with a conventional therapeutic agent, and may be administered in a single or multiple doses. It is important to administer the composition in a minimum amount which may achieve a maximum effect without any side effects, by considering all the above factors, and this will be easily determined by a person skilled in the art.

In addition, the present invention provides a method for preventing or treating age-related diseases or disorders or a method for delaying aging or extending lifespan, including administering the compound to an individual having developed or being likely to develop age-related diseases or disorders, or in need of age delay or life extension.

In addition, the present invention provides a health functional food composition for preventing or alleviating age-related diseases including: an aminoaromatic compound represented by Chemical Formula 1 or a food-scientifically acceptable salt thereof as an effective component.

The food-scientifically acceptable salt may be obtained by reacting the aminoaromatic compound with an inorganic acid such as hydrochloric acid, hydrobromic acid, sulfuric acid, nitric acid, and phosphoric acid, a sulfonic acid such as methanesulfonic acid, ethanesulfonic acid, and p-toluenesulfonic acid, or an organic carbonic acid such as tartaric acid, formic acid, citric acid, acetic acid, trichloroacetic acid, trifluoroacetic acid, capric acid, isobutyric acid, malonic acid, succinic acid, phthalic acid, gluconic acid, benzoic acid, lactic acid, fumaric acid, maleic acid, and salicylic acid. In addition, it may be obtained by reacting the aminoaromatic compound with a base to form an ammonium salt, an alkali metal salt such as sodium or potassium salt, alkaline earth metal salt such as calcium or magnesium salt, a salt of organic bases such as dicyclohexylamine, N-methyl-D-glucamine, and tris(hydroxymethyl)methylamine, and an amino acid salt such as arginine and lysine, and is not limited thereto.

The health functional food may be provided in the form of powder, granules, tablets, capsules, syrup, or beverage, and the health functional food is used with other food or food additives in addition to the aminoaromatic compound as the effective component and may be appropriately used according to a common method. The mixed amount of the effective component may be appropriately determined according to its purpose of use, for example, prevention, health, or therapeutic treatment.

The health functional food composition may include various nutritional supplements, vitamins, minerals (electrolyte), flavors such as synthetic flavors and natural flavors, colorants and enhancers (cheese, chocolate, etc.), pectic acid and salts thereof, alginic acid and salts thereof, organic acids, protective colloid thickeners, pH regulators, stabilizers, preservatives, glycerin, alcohol, carbonation agent used in carbonated drink, and the like. Other than that, fruit pulp for preparing natural fruit juice, fruit juice drink, and vegetable drink may be included. These components may be used independently or in combination.

In addition, the health functional food may further include food additives, and whether it is appropriate as "food additives" is determined by the specification and the standards for the relevant item in accordance with the general rules and general test methods of the food additive code approved by the Korea Food & Drug Administration, unless otherwise stipulated.

The items listed in the "food additive code" may include, for example, chemical synthetics such as ketones, glycine, potassium citrate, nicotinic acid, and cinnamic acid, natural additives such as persimmon pigment, licorice extract, crystalline cellulose, and guar gum, and mixed preparations such as sodium L-glutamate preparation, alkaline additives for noodles, preservative preparation, and tar colorant preparation.

The aminoaromatic compound contained in the health functional food composition may be used according to the effective dose of the pharmaceutical composition, but in the case of long-term intake for the purpose of health and hygiene or for the purpose of health control, may be used below the range, and since the effective component has no problem in terms of safety, it may be used in the amount above the range, of course.

The health functional food composition may be formulated into various formulations such as meat, sausage, bread, chocolate, candies, snacks, confectionery, pizza, ramen, other noodles, chewing gum, dairy products including ice cream, various soups, beverages, tea, drinks, alcoholic beverages, and vitamin complexes.

Hereinafter, the present invention will be described in detail through the following preferred exemplary embodiments. However, this is presented by way of illustration of the present invention, and the right scope of the present invention is not limited thereby in any sense and the right scope of the present invention is only defined by the claims set forth below.

### [Preparation Example 1] Preparation of aminoaromatic compound

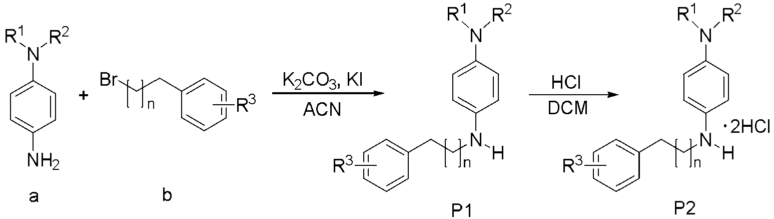

An aminoaromatic compound was prepared with reference to Korean Patent Registration No. KR 10-2643653 B1. A detailed preparation process was as follows. To an acetonitrile solution in which a *p*-phenylenediamine compound (a, 1.2 equivalent) was dissolved, potassium carbonate (3.0 equivalent), potassium iodide (0.1 equivalent), and a phenylalkyl bromide compound (b, 1.0 equivalent) were added, and the mixture was stirred at 110°C for 36 hours. Thereafter, it was cooled to room temperature, diluted with ethyl acetate, and washed with brine. The remaining organic layer was dried with Na₂SO₄, and the solvent was removed under reduced pressure. The residue was purified by a column chromatography to obtain a compound P1. The purified compound P1 was dissolved in dichloromethane (DCM), and a 4.0 M hydrogen chloride solution was added thereto. Thereafter, the solution was stirred at room temperature for 48 hours, and the produced precipitate was filtered to obtain a compound P2 in a hydrochloride form.

Aminoaromatic compounds in a hydrochloride form were prepared using the method described above, and their structures are listed in the following Table 1:

**[Table 1]**

| Example | Compound structure | Example | Compound structure |
|---|---|---|---|
| 1 | 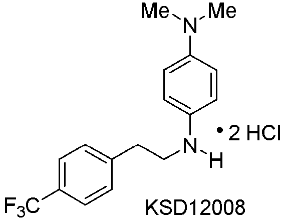 | 2 | 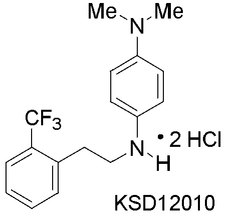 |
| 3 | 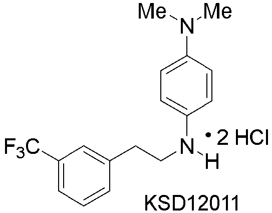 | 4 | 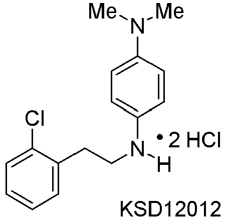 |
| 5 | 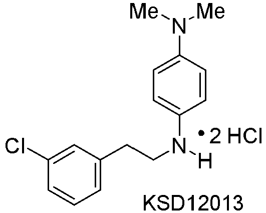 | 6 | 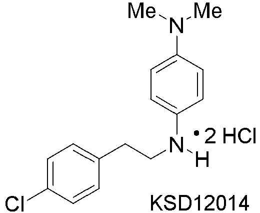 |
| 7 | 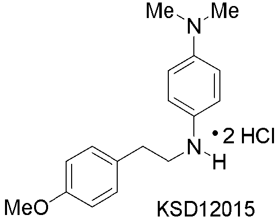 | 8 | 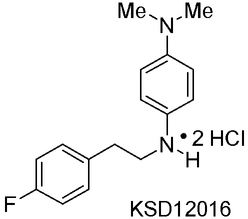 |
| 9 | 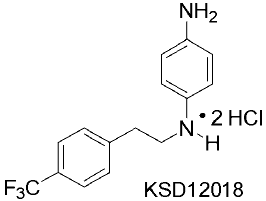 | 10 | 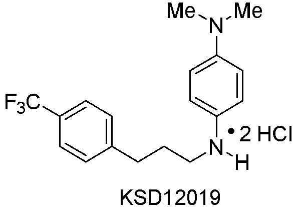 |
| 11 | 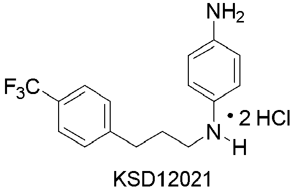 | 12 | 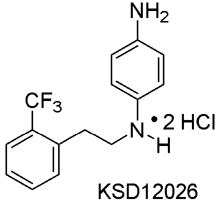 |
| 13 | 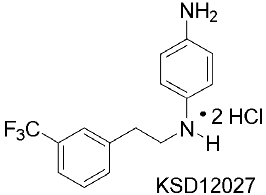 | 14 | 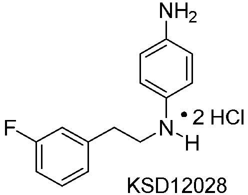 |
| 15 | 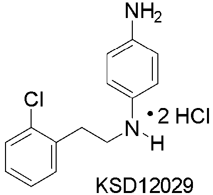 | 16 | 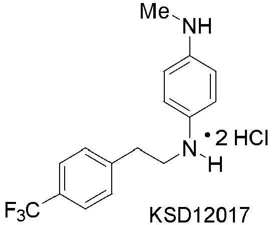 |
| 17 | 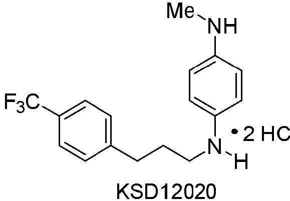 | 18 | 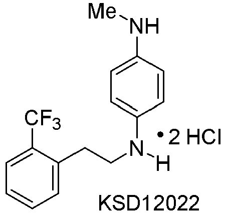 |
| 19 | 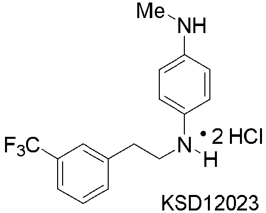 | 20 | 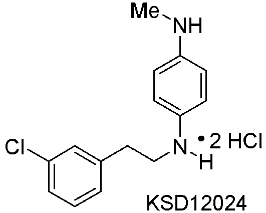 |
| 21 | 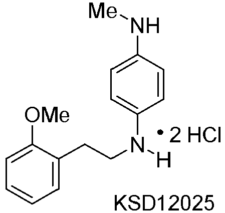 | 22 | 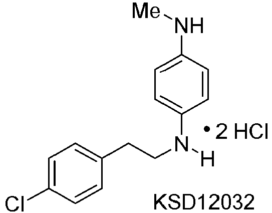 |
| 23 | 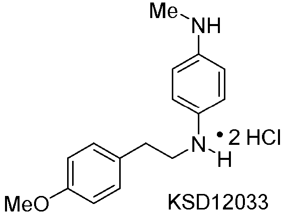 | 24 | 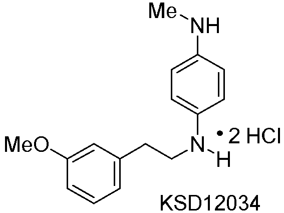 |

### Animal care and housing

One-year-old C57BL/6J experimental mice were purchased from Janvier Labs (France), and acclimated in the animal facility of Institute for Basic Science for two weeks before the experiment. The body weight of the mice was measured, and the quantity of water intake for 3 days (in grams) was monitored to calculate a drug concentration appropriate for each dosage group. The experimental mice were randomly assigned to a drug/control group and a dose group. To confirm that daily intake did not change after drug administration, water intake was monitored for 1 week. The drug concentration was adjusted every 3 months depending on changes in animal body weight and the corresponding water intake to maintain dosing. The temperature and humidity of the animal breeding room were set to 23±1°C and 50±5%, the room was maintained in a 12-hour day/night cycle (light-out at 8 p.m.), and feed and water were freely accessible. Basal body temperature and body weight were monitored every 2 weeks. Animal care and handling were performed in accordance with the guidelines of the institutional animal care and the use committee of the Institute for Basic Science.

### Quantification and Statistical Analysis

All analyses were performed in a blind manner. Data expression and statistical analysis were performed using GraphPad Prism (Graphpad Software). For image analysis, ImageJ (NIH) was used. In this analysis, a p-value less than 0.05 was regarded as being statistically significant (*P < 0.05; **P < 0.01; ***P < 0.001; ****P < 0.0001; NS, not significant).

### [Example 1] Confirmation of life extension by aminoaromatic compound

An experiment to determine a life extension degree by the aminoaromatic compound was performed using 14-month-old C57BL/6 female mice.

The experiment was conducted by dividing 14-month-old C57BL/6 female mice into 4 groups of 15 per each group. All 4 groups were fed with the same feed, but the control group was supplied with untreated general drinking water, and the remaining 3 groups were supplied with water in which an aminoaromatic compound KDS12025 was dissolved so that a daily intake was 0.1 mg per kg animal body weight (mpk), 1 mpk, and 10 mpk, respectively. The body weight and the quantity of water intake during the first 3 days of the mice were measured to determine the dissolution amount of KDS12025. Then, it was confirmed whether there were any changes to water intake in the groups provided with KDS12025 dissolved in water for a week. Later, the quantity of water intake (per day) of the mice was measured every 3 months to adjust the concentration of the drug. In addition, the body weight and the body temperature of the mice were measured every 2 weeks, the number of surviving mice was counted, and the experiment was performed for 24 months.

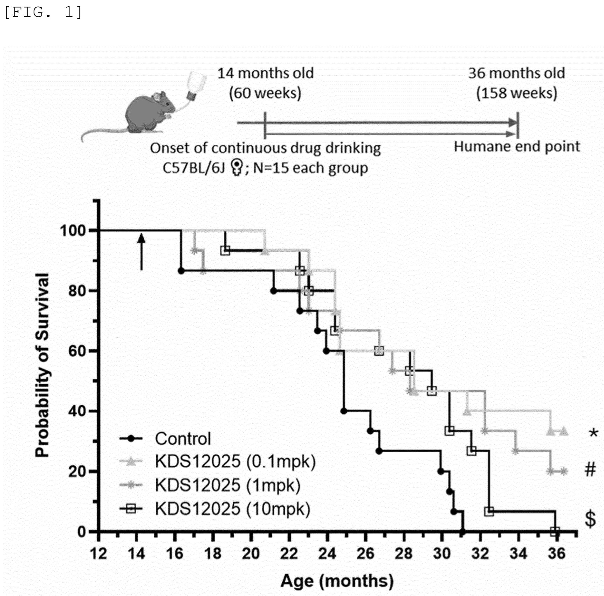

During the experiment period, the survival rate of the C57BL/6 female mice was shown in FIG. 1, and it was confirmed therefrom that the aminoaromatic compound-treated KDS12025 group had an increased survival rate as compared with the control group. In particular, it was confirmed that the survival period was statistically significantly increased in 0.1 mpk and 1 mpk of the aminoaromatic compound KDS12025-treated groups.

That is, it was confirmed that the aging of mice was delayed by the treatment with the aminoaromatic compound for a long time to improve the survival rate, resulting in life extension.

### [Example 2] Animal behavioral experiments

Animals were acclimated to a laboratory environment for 1 hour before the experiment, and the experiment was performed under ambient light and white noise. On the day of the experiment, all experiment equipment was cleaned with 10% bleach and 70% ethanol and then washed with distilled water. The behavioral experiment was performed at the same time every day. All behavioral recording was performed using an Ethovision XT (Noldus) program.

In an open field test (OFT), the animals were placed in a box having a size of 40 cm×40 cm and were allowed to explore freely for 10 minutes. The total traveled distance and speed were measured for the last 8 minutes of recording.

In a novel place recognition test (NPRT), two identical objects were placed in a box having a size of 40 cm×40 cm, and the animals were allowed to explore freely. The animals explored the object for 8 minutes and returned to the cage. After 60 minutes, one object was moved far away to another place, and the animal was put back in the experimental box. The time for an animal to explore each object was recorded for 8 minutes. The recorded video was analyzed by an experimenter who did not know the experimental conditions. The exploration was considered when the animal smells without climbing the object. A discrimination ratio was calculated as follows: (Time taken to smell moved object) / (total time taken to smell both objects).

In a Y-maze test, the animals were placed in the center of a symmetrical Y-shaped maze having three identical "arms" (length: 30 cm, height: 15 cm, width: 6 cm), allowed to explore freely, and recorded for 8 minutes. The number of times the animals entered each "arm" was recorded. An entry was considered only when all four legs of the animal were in the "arms". The alternation rate (%) was calculated as follows: Alternation rate (%) = [(total number of alternation) / (number of entries into "arms" - 2)] × 100.

In an elevated plus maze test (EPMT), the animals were placed in an open place of the symmetrical cross maze 60 cm above the ground, allowed to explore freely in the maze having two "open arms" and two "arms with wall" (length: 30 cm, width: 6 cm, wall height: 15 cm), and observed for 8 minutes. The total traveled distance, the number of entries into each "arm", and a time spent in each "arm" were recorded. An entry was considered to be successful when the center point of the animal was within the area. The time spent in the center area was not included in the time spent in each "arm".

### Results of open field test (OFT)

The experiment was conducted by dividing 14-month-old C57BL/6 female mice into 4 groups of 15 per each group. All 4 groups were fed with the same feed, but the control group was supplied with untreated general drinking water, and the remaining 3 groups were supplied with water in which an aminoaromatic compound KDS12025 was dissolved so that the daily intake was 0.1 mpk, 1 mpk, and 10 mpk, respectively. The open field test (OFT) of the C57BL/6 female mice at 26 months of age which were fed with water in which KDS12025 was dissolved for 12 months was performed. It was confirmed whether a decline in behavioral ability due to aging beyond simple life extension may also be prevented. As a comparison group, 12-month-old C57BL/6 female mice in a healthy and energetic statewere used.

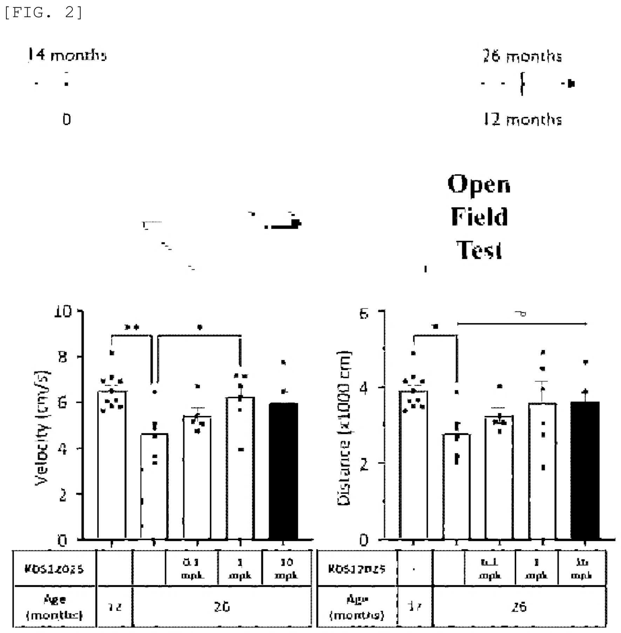

An overall degree of movement was confirmed from the results of the open field test shown in FIG. 2. It was confirmed from FIG. 2 that the average speed (left side of FIG. 2) and the total traveled distance (right side of FIG. 2) of animals aged 26 months treated with an aminoaromatic compound KDS12025 for a long time (1 year of drug administration) were statistically significantly increased and improved mobility was shown in the group treated with 1 mpk of the aminoaromatic compound KDS12025 as compared with the control group. The group treated with 0.1 and 10 mpk of the aminoaromatic compound KDS12025 had increased tendency as compared with the 26-month-old control group and no statistically significant difference as compared with the 12-month-old comparison group. The average speed and the total traveled distance showed movement and activity degree. It was confirmed that the experimental group treated with the aminoaromatic compound for a long time showed mobility equivalent to the comparison group at the age of 12 months, in spite of its age.

### Results of novel place recognition test (NPRT)

The experiment was conducted by dividing 14-month-old C57BL/6 female mice into 4 groups of 15 per each group. All 4 groups were fed with the same feed, but the control group was supplied with untreated general drinking water, and the remaining 3 groups were supplied with water in which an aminoaromatic compound KDS12025 was dissolved so that the daily intake was 0.1 mpk, 1 mpk, and 10 mpk, respectively. The novel place recognition test (NPRT) of the C57BL/6 female mice at 26 months of age which were fed with water in which KDS12025 was dissolved for 12 months was performed. As a comparison group, 12-month-old C57BL/6 female mice at a healthy and energetic time were used.

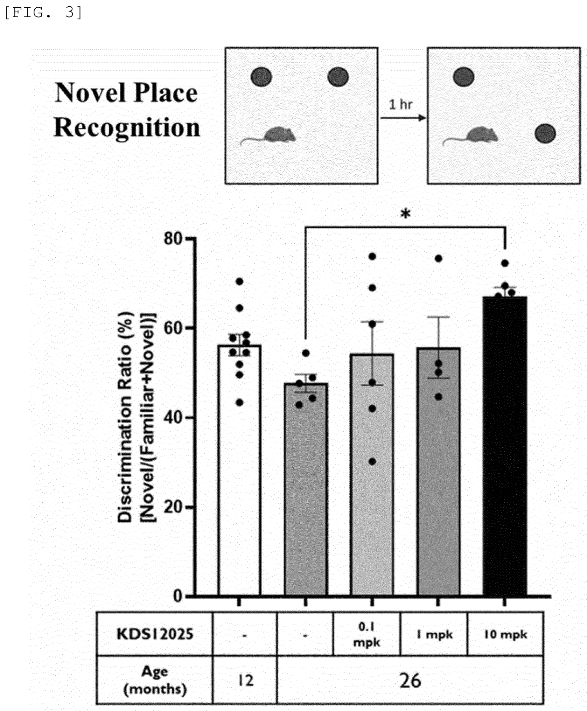

The results of novel place recognition test (NPRT) for testing memory, in particular, space-related memory are shown in FIG. 3. It was confirmed from FIG. 3 that the 26-month-old animal experimental group treated with 10 mpk of the aminoaromatic compound KDS12025 for a long time (drug administration for 1 year) showed a statistically significantly improved discrimination index for the moved object (in a new location) as compared to the control group and therefore, had improved spatial memory. Tendency with an increased discrimination index was shown in 0.1 and 1 mpk of the aminoaromatic compound KDS12025, and statistically significant difference was not shown even compared with 12-month-old mice. It was confirmed that the experimental group treated with the aminoaromatic compound for a long time showed spatial memory equivalent to or higher than the comparison group at the age of 12 months, in spite of its age.

### Results of Y-maze test

The experiment was conducted by dividing 14-month-old C57BL/6 female mice into 2 groups of 15 per each group. Both groups were fed with the same feed, but the control group was supplied with untreated general drinking water, and the remaining groups were supplied with water in which an aminoaromatic compound KDS12025 was dissolved so that the daily intake was 1 mpk. The Y-maze test of the C57BL/6 female mice at 24 months of age which were fed with water in which KDS12025 was dissolved for 10 months was performed.

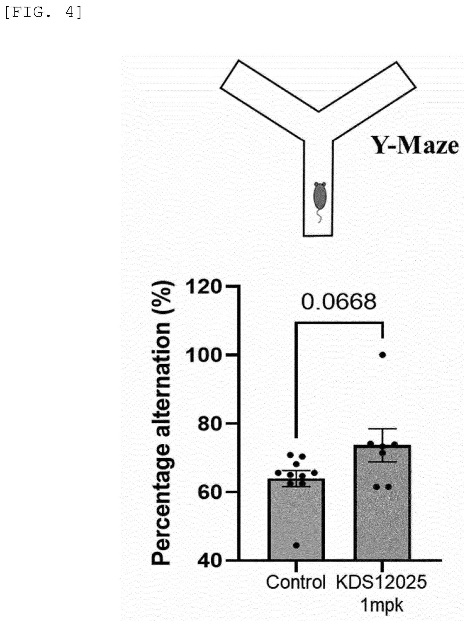

Spatial memory was evaluated by the Y-maze test, and the results are shown in FIG. 4. It was confirmed from FIG. 4 that the 24-month-old animal experimental group treated with the aminoaromatic compound KDS12025 for a long time (drug administration for 1 year) showed a statistically significantly improved alternation ratio as compared with the control group, indicating improved working memory of the older animal that consumed the compound KDS12025.

### Results of elevated plus maze (EPM) test

The experiment was conducted by dividing 14-month-old C57BL/6 female mice into 2 groups of 15 per each group. Both groups were fed with the same feed, but the control group was supplied with untreated general drinking water, and the remaining groups were supplied with water in which an aminoaromatic compound KDS12025 was dissolved so that the daily intake was 1 mpk. The elevated plus maze (EPM) test of the C57BL/6 female mice at 24 months of age which were fed with water in which KDS12025 was dissolved for 10 months was performed.

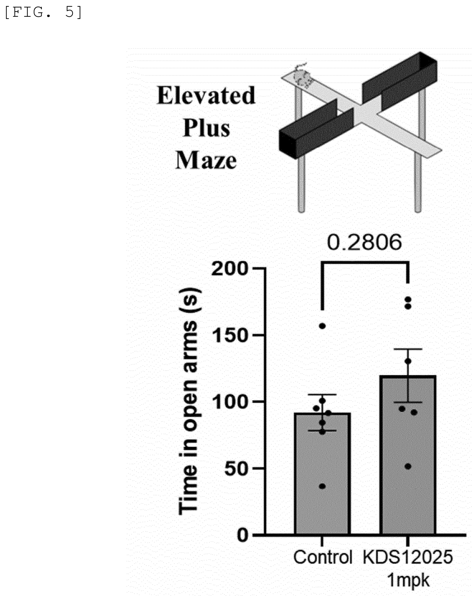

Anxiety was evaluated by the elevated plus maze (EPM) test, and the results are shown in FIG. 5. It was confirmed from FIG. 5 that the 24-month-old animal experimental group treated with the aminoaromatic compound KDS12025 for a long time (drug administration of 1 year) had an increased total time spent in open arms of the elevated plus maze as compared with the control group, which shows significantly reduced anxiety of animals in the compound-treated group.

### [Example 3] Immunohistochemical (IHC) staining of brain tissue and Sholl analysis (morphological analysis)

C57BL/6 female mice aged from 12 months to 30 months which drank water in which the aminoaromatic compound KDS12025 was dissolved so that a daily intake amount was 1 mpk were an experimental group, and C57BL/6 female mice which drank plain drinking water were a control group. 18-Month-old C57BL/6 female mice were a comparison group.

### Immunohistochemical staining

The experimental animals of each group were anesthetized with isoflurane and flushed with a 0.9% saline solution, and cool 4% paraformaldehyde (PFA) was administered thereto. Their brain was cut and fixed in 4% PFA at 4°C for a day and dehydrated in a 30% sucrose solution for 48 hours. Tissue slices having a thickness of 30 µm was prepared in a cryostat microtome and stored in a glycerol-based storage solution until the experiment. Before staining, brain slices was washed 3 times with a 0.1 M phosphate buffer, and incubated in a blocking solution (4% serum, 0.3% Triton X-100 in 0.1 M PBS) for 1 hour. A primary antibody against the target protein was added to the blocking solution at the desired dilution ratio and incubated at 4°C overnight with gentle rocking. The next day, in order to remove unbound antibodies, washing with 0.1 M PBS was performed 3 times. The corresponding secondary antibody was added, and incubation was performed at room temperature for 2 hours. In order to remove unbound secondary antibodies, washing with 0.1 M PBS was performed 3 times, and at this time, the first washing included DAPI (1:1000) for nuclear staining. The tissue slices were mounted using a fluorescent mounting medium (Dako) and dried. Fluorescent images were obtained using a Zeiss LSM900 microscope. For visualization, ZEN Digital Imaging for Light Microscopy blue system (Zeiss, ver. 3.2) and ImageJ (NIH, ver. 1.54b.) software were used.

Antibodies used in the experiment were as follows: rabbit-anti-AOC1 (aviva #ARP41908_P0050; 1:200), chicken-anti-GFAP (Millipore #AB5541; 1:500), x-anti-NeuN (1:250), rabbit-anti-TH (Pelfreez #p40101-0; 1:500), guinea pig-anti-GABA (Millipore #ab175; 1:200), mouse-anti-MAOB (SantaCruz sc-515354, 1:200), donkey-anti-chicken-647 (1:500), donkey-anti-rabbit-488 (1:200), donkey-anti-rabbit-594 (1:200), donkey-anti-guinea pig-488 (1:200), donkey-anti-mouse-594 (1:200).

### Sholl analysis (morphological analysis)

Sholl analysis was performed on a confocal microscope image according to the reference document [Nature Neuroscience volume 23, pages 1555-1566 (2020)]. The image of the brain slice immunostained with GFAP antibody was used in the Sholl analysis. Concentric circles were formed with 5 µm intervals from the center to the end of the edge of the GFAP signal, using a Sholl analysis plugin applied to ImageJ program (NIH Image, Bethesda, MD), and the number of intersections of GFAP in each circle and the radius of the largest circle crossing the astrocyte was measured and analyzed.

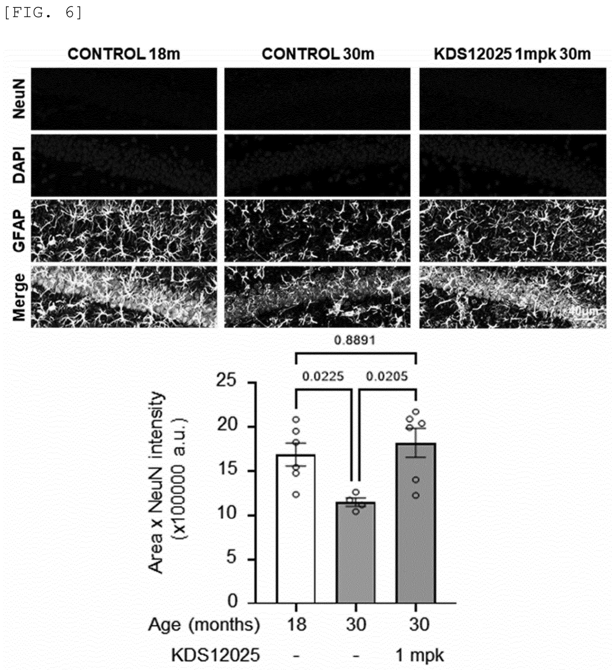

The hippocampus is an important area of the brain involved in learning and memory and is known to shrink in volume due to reduced neurogenesis and increased axonal degeneration as aging progresses. The results of measuring the degree of neuronal (NeuN) death due to aging in the hippocampus in the brain of experimental animals are shown in FIG. 6. FIG. 6 shows the degree of neuronal cell death with aging and the degree of death inhibition by the aminoaromatic compound KDS12025 with tissue staining. NeuN refers to neuron staining, DAPI refers to nuclear staining of cells, and GFAP refers to astrocyte staining. It was confirmed from FIG. 6 that the 30-month-old control group had significantly smaller pyramidal layer with lesser neurons in the hippocampal Ca21 layer as compared with the 18-month-old comparison group. However, it was confirmed that the 30-month-old experimental group statistically significantly reduced age-related neuronal cell death due by the treatment with the aminoaromatic compound KDS12025 for a long time. In particular, the experimental group treated with the aminoaromatic compound for a long time expressed neuronal cell marker NeuN at a level equal to or higher than the 18-month-old comparison group in spite of its aging and was therefore, confirmed to have delayed age-related neurodegeneration.

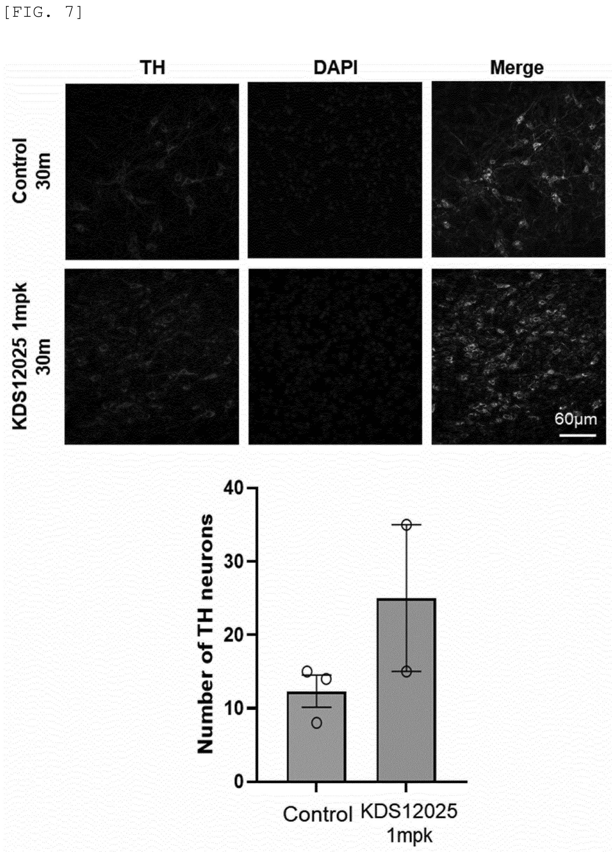

According to the previous studies, the loss of dopaminergic neurons was found in the ventral tegmental area related to a decrease in motor function in older animals. Thus, the degree of dopamine neuron loss related to behavior with aging was measured, and the results are shown in FIG. 7. FIG. 7 shows the degree of dopaminergic neuronal cell death with aging and the recovery by the aminoaromatic compound KDS12025 with tissue staining. TH refers to dopaminergic neuron staining, and DAPI refers to nuclear staining of cells. It was confirmed from FIG. 7 that in the experimental group treated with the aminoaromatic compound KDS12025 for a long time, number of dopaminergic neurons expressing tyrosine hydroxylase (TH) survived significantly. However, the control group of the same age as the experimental group had fewer TH-positive dopaminergic neurons in the ventral tegmental area and the substantia nigra. It was confirmed therefore that the experimental group treated with the aminoaromatic compound for a long time showed the significantly improved survival rate of dopaminergic neurons as compared with the control group of the same age in spite of aging.

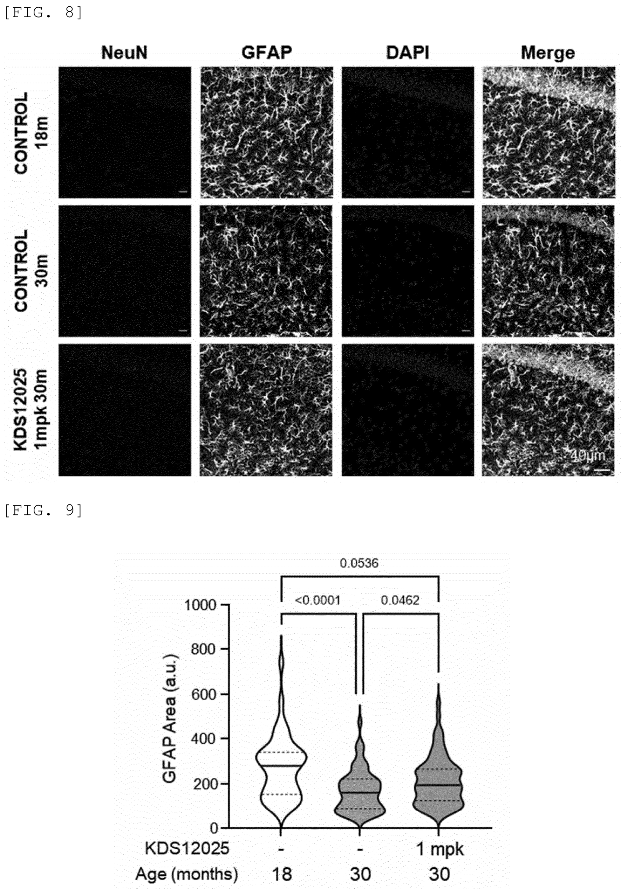

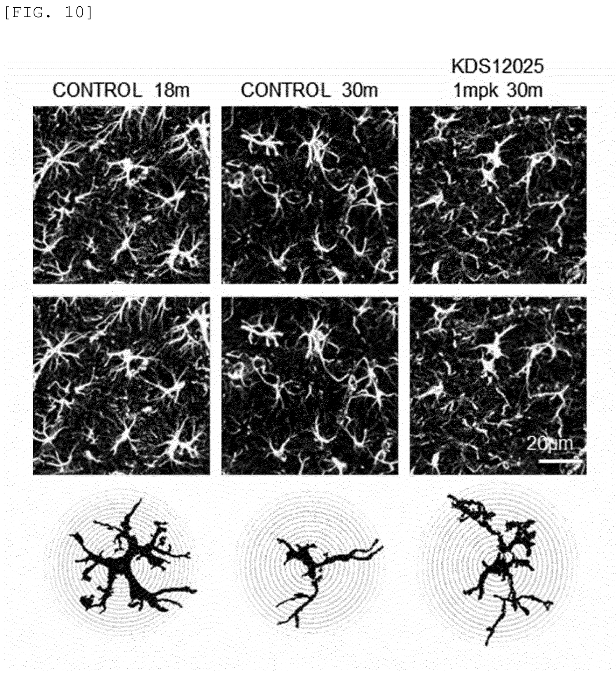

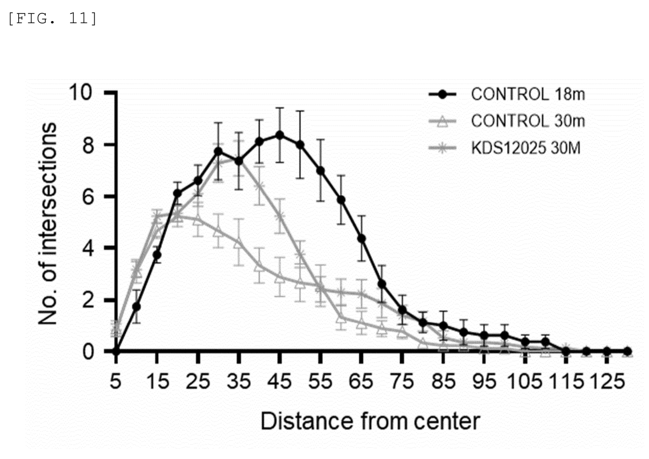

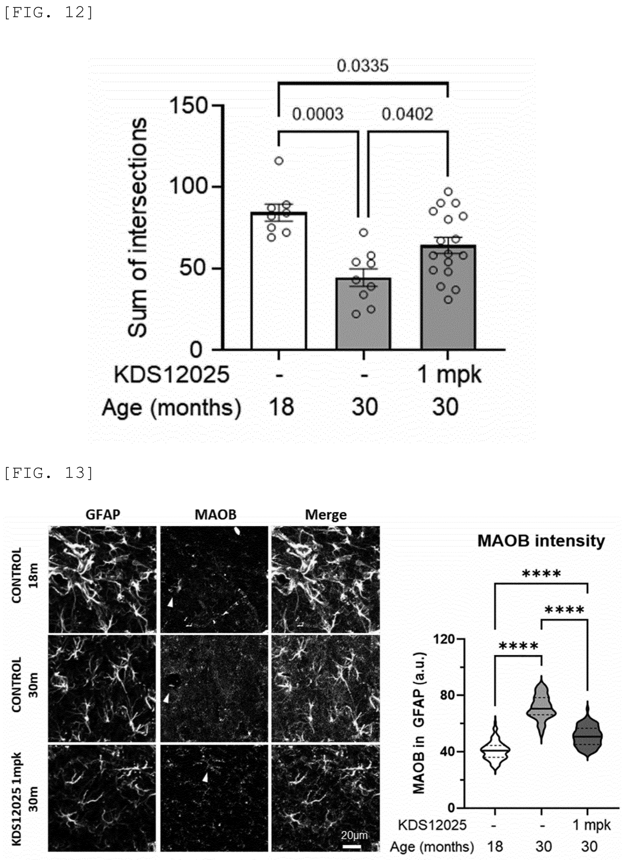

According to the previous studies, it was shown that an astrocyte area was greatly decreased due to aging, measured by decrease in the number of branches and a decrease in the volume ratio of astrocytes due to the atrophy of astrocytes (or stellate cells). The results of analyzing the external characteristics of the pathological phenomena of astrocytes (stellate cells) by immunohistochemistry and Sholl analysis are shown in FIGS. 8 to 12. FIG. 8 shows the degree of neuronal cell death with aging and the morphological characteristics of astrocytes by the aminoaromatic compound KDS12025 with tissue staining. NeuN refers to neuron staining, DAPI refers to nuclear staining of cells, and GFAP refers to astrocyte staining. FIG. 9 shows the results of measuring the size of astrocytes (GFAP area). FIG. 10 is morphological analysis photographs of astrocytes. FIGS. 11 and 12 show a part of the Sholl analysis results. FIG. 11 is a representative drawing showing the number of intersections between each circle and cells starting from the center of the cell, during the Sholl analysis, and normal cells were branched and had many intersections, but aged cells atrophied had fewer intersections. FIG. 12 is a drawing showing the sum of the numbers of intersections between branches and circles of the Sholl analysis in order to measure the morphological characteristics of the astrocytes, where more intersections mean more branching.

It was confirmed that the 30-month-old control group had decreased astrocyte area as compared with the 18-month-old comparison group due to aging and also had a decreased astrocyte branching measured in the Sholl analysis of individual cells. Surprisingly, though the astrocytes atrophied much due to aging in the control group, the experimental group of the same age treated with the aminoaromatic compound KDS12025 for a long time displayed size and the number of branches of the astrocytes to the level equivalent to or higher than the 18-month-old comparison group despite the chronological age (FIGS. 9 to 12). It was confirmed therefrom that the size and the number of branches of the astrocytes tended to decrease as aging progressed, but may be delayed by administering the aminoaromatic compound.

In addition, the results of analyzing the expression pattern of MAOB protein which is one of the mechanisms of pathological phenomena in astrocytes are shown in FIG. 13. GFAP refers to astrocyte staining, and MAOB refers to MAOB protein staining. It was confirmed therefrom that the 30-month-old control group overproduced MAOB in the astrocytes due to aging, but the experimental group of the same age to which the aminoaromatic compound KDS12025 was administered for a long time had lower levels of MAOB. It was confirmed that production of MAOB in astrocytes was decreased despite the progression of animal aging, by administering the aminoaromatic compound for a long time, and therefore, overproduction of reactive oxygen and GABA causing memory loss or cognitive impairment was inhibited.

## Claims

1. A pharmaceutical composition for preventing or treating age-related diseases or disorders comprising: an aminoaromatic compound represented by the following Chemical Formula 1 or a pharmaceutically acceptable salt thereof as an effective component: 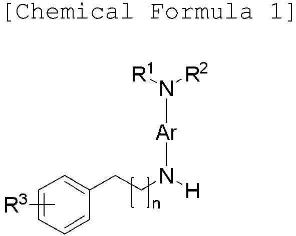 wherein
Ar is C₆-C₂₀ arylene, and the arylene of Ar may be further substituted by one or more selected from C₁-C₁₀ alkyl, C₁-C₁₀ alkoxy, amino, mono- or di-C₁-C₁₀ alkylamino, haloC₁-C₁₀ alkyl, haloC₁-C₁₀ alkoxy, and hydroxy;
R¹ and R² are independently of each other hydrogen or C₁-C₁₀ alkyl;
R³ is halogen, C₁-C₁₀ alkoxy, haloC₁-C₁₀ alkyl, or haloC₁-C₁₀ alkoxy; and
n is an integer of 1 or 2,
provided that when R³ is halogen, n is an integer of 1.

2. The pharmaceutical composition of claim 1, wherein Ar is phenylene or biphenylene; and may be further substituted by one or more selected from C₁-C₇ alkyl, C₁-C₇ alkoxy, amino, and hydroxy.

3. The pharmaceutical composition of claim 1, wherein the aminoaromatic compound is represented by the following Chemical Formula 2: 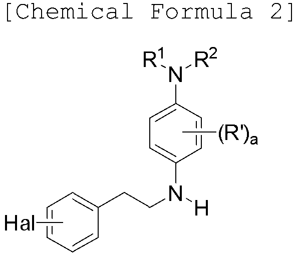 wherein
R¹ and R² are independently of each other hydrogen or C₁-C₇ alkyl;
Hal is halogen;
R' is C₁-C₇ alkyl, C₁-C₇ alkoxy, amino, or hydroxy; and
a is an integer of 0 to 4.

4. The pharmaceutical composition of claim 1, wherein the aminoaromatic compound is represented by the following Chemical Formula 3: 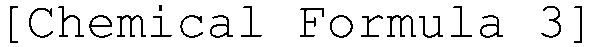 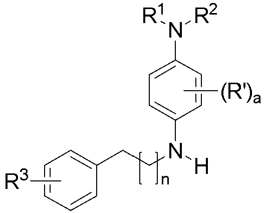 wherein
R¹ and R² are independently of each other hydrogen or C₁-C₇ alkyl;
R³ is C₁-C₇ alkoxy or haloC₁-C₇ alkyl;
R' is C₁-C₇ alkyl, C₁-C₇ alkoxy, amino, or hydroxy;
a is an integer of 0 to 4; and
n is an integer of 1 or 2.

5. The pharmaceutical composition of claim 3, wherein R¹ and R² are independently of each other hydrogen or C₁-C₄ alkyl; Hal is halogen; and a is an integer of 0.

6. The pharmaceutical composition of claim 4, wherein R¹ and R² are independently of each other hydrogen or C₁-C₄ alkyl; R³ is C₁-C₄ alkoxy or haloC₁-C₄ alkyl; a is an integer of 0; and n is an integer of 1 or 2.

7. The pharmaceutical composition of claim 3, wherein the aminoaromatic compound is any one selected from the following compound group:

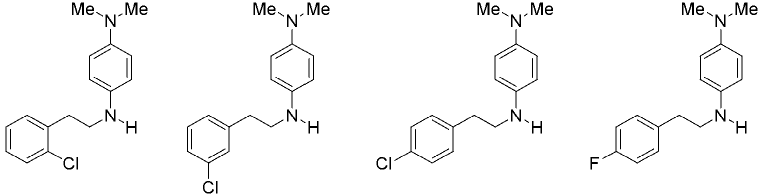

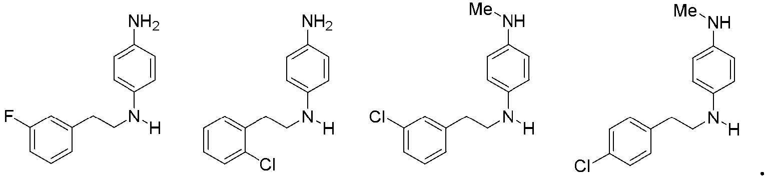

8. The pharmaceutical composition of claim 4, wherein the aminoaromatic compound is any one selected from the following compound group:

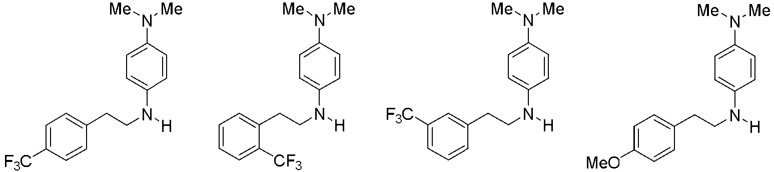

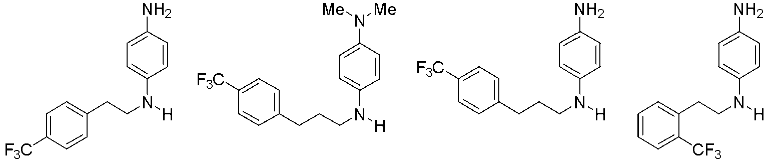

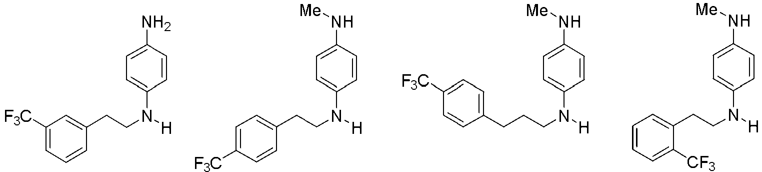

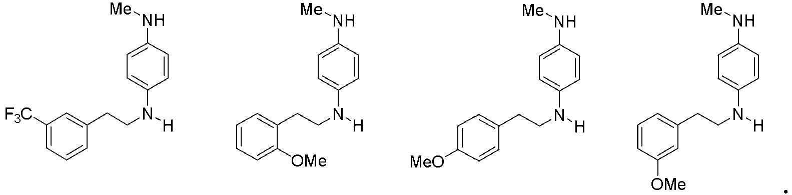

9. The pharmaceutical composition of claim 1, wherein the pharmaceutically acceptable salt is a hydrochloride.

10. The pharmaceutical composition of claim 1, wherein the age-related disease is one or more diseases selected from the group consisting of Alzheimer's disease, diabetes, Parkinson's disease, Huntington's disease, degenerative joint disease, stroke, angina pectoris, osteoporosis, myocardial infarction, muscular dystrophy, amyotrophic lateral sclerosis, sarcopenia, liver cirrhosis, and chronic kidney disease.

11. The pharmaceutical composition of claim 1, wherein the age-related disease is a cognitive dysfunction disease due to aging.

12. The pharmaceutical composition of claim 11, wherein the cognitive function is one or more selected from the group consisting of perception, memory, attention, speech comprehension, speech generation, reading comprehension, creation of imagery, learning, and reasoning.

13. A composition for delaying aging or extending lifespan comprising: an aminoaromatic compound represented by Chemical Formula 1 or a pharmaceutically acceptable salt thereof as an effective component: 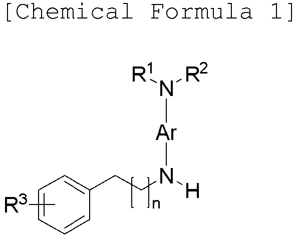 wherein
Ar is C₆-C₂₀ arylene, and the arylene of Ar may be further substituted by one or more selected from C₁-C₁₀ alkyl, C₁-C₁₀ alkoxy, amino, mono- or di-C₁-C₁₀ alkylamino, haloC₁-C₁₀ alkyl, haloC₁-C₁₀ alkoxy, and hydroxy;
R¹ and R² are independently of each other hydrogen or C₁-C₁₀ alkyl;
R³ is halogen, C₁-C₁₀ alkoxy, haloC₁-C₁₀ alkyl, or haloC₁-C₁₀ alkoxy; and
n is an integer of 1 or 2,
provided that when R³ is halogen, n is an integer of 1.

14. A health functional food composition for preventing or alleviating age-related diseases comprising: an aminoaromatic compound represented by Chemical Formula 1 or a food-scientifically acceptable salt thereof as an effective component: 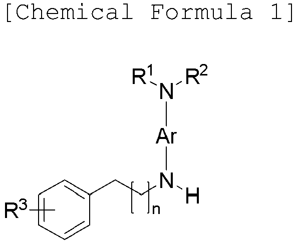 wherein
Ar is C₆-C₂₀ arylene, and the arylene of Ar may be further substituted by one or more selected from C₁-C₁₀ alkyl, C₁-C₁₀ alkoxy, amino, mono- or di-C₁-C₁₀ alkylamino, haloC₁-C₁₀ alkyl, haloC₁-C₁₀ alkoxy, and hydroxy;
R¹ and R² are independently of each other hydrogen or C₁-C₁₀ alkyl;
R³ is halogen, C₁-C₁₀ alkoxy, haloC₁-C₁₀ alkyl, or haloC₁-C₁₀ alkoxy; and
n is an integer of 1 or 2,
provided that when R³ is halogen, n is an integer of 1.
